# EUROPEAN PATENT APPLICATION

(11) **EP 4 696 708 A1**
(43) Date of publication of application: **18.02.2026**
(21) Application number: 24787948.9
(22) Date of filing: 02.04.2024
(51) Int. Cl.: C07K 14/575, A61K 38/22, A61P 3/04

(54) **PEPTIDE TYROSINE-TYROSINE ANALOG AND USE THEREOF**

(30) Priority: 10.04.2023 CN 202310372497
(71) Applicant: The United Bio-Technology (Hengqin) Co., Ltd., Zhuhai, Guangdong 519031 (CN)
(72) Inventor: HUANG, Liang, Zhuhai, Guangdong 519031 (CN); LIU, Xiaoxiao, Zhuhai, Guangdong 519031 (CN); CAO, Chunlai, Zhuhai, Guangdong 519031 (CN); ZHOU, Cui, Zhuhai, Guangdong 519031 (CN); HE, Xiuyi, Zhuhai, Guangdong 519031 (CN); DENG, Huixing, Zhuhai, Guangdong 519031 (CN); XIE, Xin, Zhuhai, Guangdong 519031 (CN)
(74) Representative: Michalski Hüttermann & Partner Patentanwälte mbB
(86) International application number: PCT/CN2024/085499
(87) International publication number: WO 2024/212843

(57) **Abstract**

The present application relates to the pharmaceutical field, and specifically provides a novel peptide tyrosine-tyrosine (PYY₃₋₃₆) analogue or its salt or solvate, comprising a sequence structure of the following general formula (I):

P-K-P-E-ψ-P-E-X₁₀-D-X₁₂-S-P-E-E-W-Q-R-Y-Y-X₂₂-X₂₃-L-R-H-Y-L-N- W-L-T-R-Q-R-Y-R₁ (I).

The novel peptide YY analogue (PYY₃₋₃₆) provided by the present application has better pharmacological effects, longer action time, excellent bioavailability and safety.

## Description

### TECHNICAL FIELD

The present application relates to the field of biomedicine, and more specifically relates to novel peptide tyrosine-tyrosine analogue and use thereof.

### BACKGROUND ART

Peptide tyrosine-tyrosine (PYY) is a 36-amino-acid intestinal hormone primarily synthesized and secreted by intestinal L cells (Spreckley E, Murphy KG. Front Nutr. 2015; 2:23). There are two native forms of human PYY: PYY1-36 and PYY3-36. PYY3-36 is the main circulation form, produced by cleaving the N-terminus of PYY1-36 by dipeptidyl peptidase-4 (DPP-4). PYY3-36 has Y2 selectivity and can inhibit the release of NPY by activating Y2 receptors on NPY containing cells in the arcuate nucleus, thereby reducing appetite and promoting weight loss.

PYY3-36 can activate NPY2 receptors, and reduce appetite. It has been currently studied as a potential therapeutic drug for weight regulation, especially for the treatment of obesity and obesity complications. However, there is still a need for PYY3-36 analogs with enhanced NPY2 receptor selectivity and excitatory activity.

Due to protease and other clearance mechanisms, the half-life of exogenous PYY3-36 is approximately 10-15 minutes. Due to its short half-life, it needs to be administered at least once a day to exert its therapeutic effect. Therefore, it is necessary to increase the half-life of PYY3-36, such as by adding fatty acid side chains to PYY3-36 analogues to increase its half-life.

Despite the continuous deepening of research on the role of PYY3-36 in metabolism, there is still a need for better PYY3-36 analogues, especially those with better NPY2 receptor activity and selectivity, stronger in vivo drug effect, and longer half-life.

### SUMMARY

The present application aims to provide a novel Peptide tyrosine-tyrosine (PYY₃₋₃₆) analogue or its salt or solvate. More specifically, this novel Peptide tyrosine-tyrosine (PYY₃₋₃₆) analogue has better pharmacological efficacy, longer action time, excellent bioavailability and safety.

In one aspect, the present application provides a novel Peptide tyrosine-tyrosine (PYY₃₋₃₆) analogue or its salt or solvate, comprising a sequence structure of the following general formula (I):

P-K-P-E-ψ-P-E-X₁₀-D-X₁₂-S-P-E-E-W-Q-R-Y-Y-X₂₂-X₂₃-L-R-H-Y-L-N-W-L-T-R-Q- R-Y-R₁ (I),

wherein ψ is Lys whose side chain is modified with the structure of the following general formula (II):
Y-Z (II), wherein Y is (AEEAc or Glu)ₐ-(AEEAc or Glu)_{b}-(AEEAc or Glu)_{c}, wherein a, b, and c are each independently 0 or 1, and a, b, and c are not all 0 at the same time (as an exemplary illustration, it can be AEEAc-AEEAc-γGlu), the carboxyl end of Y is connected to the ε-amino group of the side chain of Lys, Z is-CO-(CH₂)ₘ-COOH, and m is an integer between 6-24;
X₁₀ is E or Aib;
X₁₂ is A or Aib;
X₂₂ is A or Aib;
X₂₃ is S or E;
R₁ is NH₂ or OH;
and at least one of X₁₀, X₁₂, and X₂₂ is Aib.

In the present application, as one of the embodiments, Y in the general formula (II) is AEEAc-γGlu or AEEAc-AEEAc-γGlu, preferably AEEAc-γGlu.

In the present application, as one of the embodiments, the analogue is preferably selected from:

PKPEK(AEEAc-yGlu-CO(CH₂)₁₆COOH)PEED-Aib-SPEEWQRYY -Aib-SLRHYLNWLT RQRY-NH₂,

PKPEK(AEEAc-yGlu-CO(CH₂)₁₆COOH)PE-Aib-DASPEEWQRYYAELRHYLNWLTRQ RY-NH₂, or

PKPEK(AEEAc-yGlu-CO(CH₂)₁₆COOH)PE-Aib-DASPEEWQRYY-Aib-ELRHYLNWLT RQRY-NH₂.

In the present application, as one of the embodiments, the analogue is more preferably selected from:

PKPEK(AEEAc-γGlu-CO(CH₂)₁₆COOH)PE-Aib-DASPEEWQRYY-Aib-ELRHYLN WLTRQRY-NH₂.

The analogues described in the present application are chemically modified by fatty acid side chain groups at specific site X₇ of peptide skeleton, and at least one or more non-natural amino acids Aib are introduced at the specific site of skeleton. The above modification enables the peptide chains of the analogues to have a more stable peptide alpha helix structure and enhanced albumin binding capacity, thereby improving the stability of the peptide analogues, enhancing in vivo drug effect, and prolonging action time of the peptide.

The present application provides a pharmaceutical composition including an effective amount of any of the above analogues or its salts or solvates, and pharmaceutically acceptable excipient, diluent, carriers or pharmaceutic adjuvant.

In the present application, as one of the embodiments, the pharmaceutical composition is in the form of an injection or lyophilized powder, tablet, pill, troche, soft capsule, hard capsule, granule, powder, solution, microneedle, suspension or syrup; and as one of the embodiments, the pharmaceutical composition is in the form of microcapsule, microsphere, nanoparticle, or liposome.

In the present application, as one of the embodiments, the pharmaceutical composition is used for oral administration, inhalation administration, transdermal administration, or parenteral administration; and as one of the embodiments, the parenteral administration is selected from intraperitoneal, intramuscular, intra-arterial, intravenous, subcutaneous or intradermal injection administration.

In the present application, as one of the embodiments, the pharmaceutical composition is administered at a frequency of at least once a day or once a week.

In one aspect, the use of an analogue or salt or solvate thereof as described in any of the aforementioned aspects in the preparation of a drug for the treatment, prevention, or reduction of overweight and obesity or obesity related complications is provided. As one of the embodiments, the obesity complication disease refers to any disease or disorder caused or aggravated by obesity, including but not limited to angina pectoris, cardiovascular disease, cholecystitis, cholelithiasis, congestive heart-failure, heart failure with preserved ejection fraction, dyslipidemia, non-alcoholic steatohepatitis, fertility complications, glucose intolerance, gout, hypertension, hypothyroidism, hyperinsulinemia, insulin resistance, osteoarthritis, polycystic ovary syndrome, pregnancy complications, psychological disorder, sleep apnea and other respiratory problems, stress urinary incontinence apoplexy, type II diabetes, uric acid kidney stones, mammary cancer, colon cancer, carcinoma of endometrium, esophageal cancer, carcinoma of gallbladder, carcinoma of kidney, prostate cancer or rectal cancer.

In one aspect, the pharmaceutical composition can be used in combination with other drugs for treating the same or related diseases, including but not limited to one, two or more of metformin, sulfonylurea, SGLT-1/2 inhibitors, DPP-4 inhibitors, insulins, GLP-1, GCG, GIP, and FGF-21.

The term "overweight" or "obesity" as described in the present application refers to the situation where an individual's body mass index (BMI) exceeds a specific standard. This standard varies in numerical values depending on different races. For example, reference can be made to the "Overweight & Obesity" of the US Centers for Disease Control and Prevention and the "Definitions & Facts for Adult Overweight & Obesity" of the US National Institutes of Health. If the BMI of Americans is more than 30 kg/m², they can be considered obese. If the BMI is 25.0 kg/m² to 30 kg/m², they can be considered overweight. According to the standards of the National Health Commission of the People's Republic of China, Chinese people are overweight if their BMI is 24.0 kg/m² to 27.9 kg/m², and obese if their BMI is ≥ 28.0 kg/m².

The term "PYY" as described in the present application means peptide tyrosine-tyrosine derived from any species. PYY includes natural PYY (i.e. sequence 1-36, full-length) and its variants (i.e. natural PYY additions, deletions, and/or substitutions), with specific PYY including but not limited to natural human PYY₁₋₃₆ and natural human PYY₃₋₃₆.

The term "PYY₃₋₃₆ analogue" as described in the present application means a PYY₃₋₃₆ like peptide or polypeptide that induces one or more NPY receptors (such as NPY2 receptors) to produce excitatory activity similar to that induced by natural PYY₃₋₃₆. In some cases, when compared to natural human PYY₃₋₃₆, the PYY₃₋₃₆ analogues mentioned herein can bind to NPY receptors such as NPY2 receptors with higher or lower affinity, while demonstrating longer in vivo or in vitro half-lives. Therefore, the PYY₃₋₃₆ analogues mentioned herein are synthetic analogues that act as NPY2 receptor agonists.

The "excitatory activity" induced by PYY₃₋₃₆ analogues on NPY receptors as described in the present application refers to the ability of analogues to excite specific NPY receptors (such as NPY2 receptors) cells to produce cAMP. The cells used can be host cells constructed by a person skilled in the art that overexpress specific NPY receptors. The excitatory activity of receptors can be measured using the EC₅₀ value of cAMP produced by receptor cells stimulated by analogues. The EC₅₀ value refers to the drug concentration required to achieve half of the maximum activity (50% activity) of an analogue in a specific assay system.

The term "treatment" as described in the present application means to weaken, reverse, slow down, or stop the progression or severity of an existing condition, disease, or symptom.

The specific meanings of abbreviations used in the present application are as follows:
MBHA: 4-Toluene Hydroamine
DMF: N,N-dimethyl formamide
Aib: α-amino isobutyric acid
AEEAc: [2-(2-amino-ethoxy)-ethoxy]-acetyl
cAMP: cyclic adenosine monophosphate
NPY2R: Neuropeptide Y receptor 2
NPY1R: Neuropeptide Y receptor 1
NPY5R: Neuropeptide Y receptor 5
HEK-293: Human embryonic kidney cells 293
PBS: Phosphate buffered saline
Forskolin: Forskolin
HBSS: Hank's balanced salt solution
HEPES: (4- (2-hydroxyethyl) -1-piperazine ethanesulfonic acid) buffer solution
IBMX: 3-isobutyl-1-methylxanthine
EC₅₀: Median effect concentration
DPBS: Dulbecco's phosphate-buffered saline
EDTA: Ethylene diamine tetraacetic acid
FLIPR: Fluorescence imaging plate reader
LC-MS/MS: Liquid chromatography-mass spectrometry/mass spectrometry hyphenated techniques

### BRIEF DESCRIPTION OF THE DRAWINGS

FIG. 1a. The effect of Compound 1, Compound 2, Compound 3, and Reference Example (Ref.) 1 on the accumulation of food intake inhibition in normal mice after a single injection. *P<0.05, **P<0.01, ***P<0.001 vs Vehicle (solvent)# P<0.05, ##P<0.01, ###P<0.001 vs Ref.1-3 nmol/kg, ^P<0.05, ^^P<0.01, ^^^P<0.001 vs Ref.1-10 nmol/kg, ^{&}P<0.05 vs Ref.1-30 nmol/kg;
FIG. 1b. The effect of Compound 1, Compound 2, Compound 3, and Reference Example 1 on weight loss in normal mice after a single injection. *P<0.05, **P<0.01, ***P<0.001 vs Vehicle (solvent), ^{&}P<0.05 vs Ref. 1-30 nmol/kg.
FIG. 2: The effect of combination therapy with Compound 3 and GLP-1 agonist Semaglutide on weight loss in normal SD rats. *P<0.05, **P<0.01, ***P<0.001 vs Vehicle (solvent), # P<0.05, ##P<0.01, vs Semaglutide 20 nmol/kg.

### DETAILED DESCRIPTION

The present application is further described below in details through the following examples. These examples are for illustrative purposes only, not intended to limit the scope of the present application.

### Example 1: Synthesis of Analogues

The analogues in the table below were synthesized through chemical synthesis methods.

**Table 1.1 List of PYY₃₋₃₆ analogues and their molecular weight and purity**

| Name | Molecular Formula | Theoretical Molecular Weight | Purity |
|---|---|---|---|
| PYY (SEQ ID NO:1) | YPIKPEAPGEDASPEELNRYYASL RHYLNLVTRQRY-NH2 | 4309.75 | 97.44% |
| PYY₃₋₃₆ (SEQ ID NO:2) | IKPEAPGEDASPEELNRYYASLR HYLNLVTRQRY-NH2 | 4049.46 | 97.16% |
| Analogue 1 (SEQ ID NO:3) | PKPEK(AEEAc-γGlu-CO(CH2)16CO OH)PEED-Aib-SPEEWQRYY-Aib- SLRHYLNWLTRQRY-NH2 | 4935.59 | 97.73% |
| Analogue 2 (SEQ ID NO:4) | PKPEK(AEEAc-γGlu-CO(CH2)16CO OH)PE-Aib-DASPEEWQRYYAEL RHYLNWLTRQRY-NH2 | 4905.56 | 95.99% |
| Analogue 3 | PKPEK(AEEAc-γGlu-CO(CH₂)₁₆CO | 4919.59 | 95.55% |
| (SEQ ID NO:5) | OH)PE-Aib-DASPEEWQRYY-Aib- ELRHYLNWLTRQRY-NH2 | | |
| Reference Example 1 (SEQ ID NO:6) | PKPEK(AEEAc-AEEAc-γGlu-CO(C H2)16COOH)PGKDASPEEWNRYY ADLRHYLNWLTRQRY-NH2 | 4993.67 | 95.24% |

Note:
Reference Example 1 was selected from Example 4 of U.S. Patent Application No. US20200140514A1
Y of X36 in SEQ ID NO: 1 and SEQ ID NO: 2 was amidated;
K of X7 in SEQ ID NO: 3- SEQ ID NO: 5 passed through the fatty acid side chain group;
AEEAc-γGlu-CO (CH₂)₁₆COOH modified the K side chain, and the Y of X36 is amidated;
In SEQ ID NO: 6, X7 modified the K side chain through the fatty acid side chain group AEEAc-AEEAc-γGlu-CO(CH₂)₁₆COOH, and Y of X36 was amidated.

### Analogue 1

PKPEK(AEEA-yGlu-CO-(CH₂)₁₆-COOH)PEED-Aib-SPEEWQRYY-Aib-SLRHYLN WLTRQRY-NH₂

Analogue 1 was synthesized using standard Fmoc chemical method.
1) Resin preparation: MBHA resin (0.28 mmol, 1.00 eq, Sub 0.28 mmol/g) was added with a certain amount of DMF and stirred under N₂ at 20°C for 2 hours. The mixture was filtered to obtain the desired resin.
2) Deprotection: 15.0 mL of DMF containing 20% piperidine was added to the above resin. The resin was stirred under N₂ for 15 minutes, washed with DMF (15.0 mL*5) and filtered to obtain the deprotected resin.
3) Coupling: Fmoc-Tyr (tBu)-OH (3.00 eq), DIEA (6.00 eq), and HBTU (2.85 eq) were dissolved in DMF (4.00 mL) to prepare a solution, added with the resin obtained in step 2), and stirred under N₂ at 20°C for 20 minutes. The resin was rinsed with DMF (15.0 mL*5).
4) Steps 2)-3) were repeated, a specified amount of raw materials were added in the order listed in the table below, and the following amino acids were coupled to synthesize peptide chains:

| **#** | **Raw material** | **Coupling reagents** |
|---|---|---|
| 2 | Fmoc-Arg(Pbf)-OH (3.00 eq) | HATU (2.85 eq) and DIEA (6.00 eq) |
| 3 | Fmoc-Gln(Trt)-OH (3.00 eq) | HATU (2.85 eq) and DIEA (6.00 eq) |
| 4 | Fmoc-Arg(Pbf)-OH (3.00 eq) | HATU (2.85 eq) and DIEA (6.00 eq) |
| 5 | Fmoc-Thr(tBu)-OH (3.00 eq) | HATU (2.85 eq) and DIEA (6.00 eq) |
| 6 | Fmoc-Leu-OH (3.00 eq) | DIC (3.00 eq) and HOAT (3.00 eq) |
| 7 | Fmoc-Trp(Boc)-OH (3.00 eq) | HATU (2.85 eq) and DIEA (6.00 eq) |
| 8 | Fmoc-Asn(Trt)-OH (3.00 eq) | HATU (2.85 eq) and DIEA (6.00 eq) |
| 9 | Fmoc-Leu-OH (3.00 eq) | HATU (2.85 eq) and DIEA (6.00 eq) |
| 10 | Fmoc-Tyr(tBu)-OH (3.00 eq) | DIC (3.00 eq) and HOAT (3.00 eq) |
| 11 | Fmoc-His(Trt) -OH(3.00 eq) | HATU (2.85 eq) and DIEA (6.00 eq) |
| 12 | Fmoc-Arg(Pbf)-OH (3.00 eq) | HATU (2.85 eq) and DIEA (6.00 eq) |
| 13 | Fmoc-Leu-OH (3.00 eq) | HATU (2.85 eq) and DIEA (6.00 eq) |
| 14 | Fmoc-Ser(tBu)-OH (3.00 eq) | HATU (2.85 eq) and DIEA (6.00 eq) |
| 15 | Fmoc-Aib-OH (3.00 eq) | HATU (2.85 eq) and DIEA (6.00 eq) |
| 16 | Fmoc-Tyr(tBu)-OH (3.00 eq) | DIC (3.00 eq) and HOAT (3.00 eq) |
| 17 | Fmoc-Tyr(tBu)-OH (3.00 eq) | HATU (2.85 eq) and DIEA (6.00 eq) |
| 18 | Fmoc-Arg(Pbf)-OH (3.00 eq) | HATU (2.85 eq) and DIEA (6.00 eq) |
| 19 | Fmoc-Gln(Trt)-OH (3.00 eq) | HATU (2.85 eq) and DIEA (6.00 eq) |
| 20 | Fmoc-Trp(Boc)-OH (3.00 eq) | HATU (2.85 eq) and DIEA (6.00 eq) |
| 21 | Fmoc-Glu(OtBu)-OH (3.00 eq) | HATU (2.85 eq) and DIEA (6.00 eq) |
| 22 | Fmoc-Glu(OtBu)-OH (3.00 eq) | DIC (3.00 eq) and HOAT (3.00 eq) |
| 23 | Fmoc-Pro-OH (3.00 eq) | HATU (2.85 eq) and DIEA (6.00 eq) |
| 24 | Fmoc-Ser(tBu)-OH (3.00 eq) | HATU (2.85 eq) and DIEA (6.00 eq) |
| 25 | Fmoc-Ala-OH (3.00 eq) | HATU (2.85 eq) and DIEA (6.00 eq) |
| 26 | Fmoc-Asp(OtBu)-OH (3.00 eq) | HATU (2.85 eq) and DIEA (6.00 eq) |
| 27 | Fmoc-Aib-OH (3.00 eq) | HATU (2.85 eq) and DIEA (6.00 eq) |
| 28 | Fmoc-Asp(OtBu)-OH (3.00 eq) | HATU (2.85 eq) and DIEA (6.00 eq) |
| 29 | Fmoc-Glu(OtBu)-OH (3.00 eq) | DIC (3.00 eq) and HOAT (3.00 eq) |
| 30 | Fmoc-Glu(OtBu)-OH (3.00 eq) | DIC (3.00 eq) and HOAT (3.00 eq) |
| 31 | Fmoc-Pro-OH (3.00 eq) | DIC (3.00 eq) and HOAT (3.00 eq) |
| 32 | Fmoc-Lys(Dde) -OH (3.00 eq) | HATU (2.85 eq) and DIEA (6.00 eq) |
| 33 | Fmoc-Glu(OtBu)-OH (3.00 eq) | HATU (2.85 eq) and DIEA (6.00 eq) |
| 32 | Fmoc-Pro-OH (3.00 eq) | HATU (2.85 eq) and DIEA (6.00 eq) |
| 33 | Fmoc-Lys(Boc) -OH (3.00 eq) | HATU (2.85 eq) and DIEA (6.00 eq) |
| 34 | Boc-Pro-OH (3.00 eq) | DIC (3.00 eq) and HOAT (3.00 eq) |

5) 3% N₂H₄·H₂O/DMF solution was added to the above reaction system, and allowed to react twice under N₂ for 30 minutes each time. Rinsing was performed for 5 times with DMF.
6) Steps 2-3) were repeated, and the raw materials were added in the order listed in the table below, and the following groups were coupled and the peptide chain was modified with fatty acid side chains:

| | | |
|---|---|---|
| 35 | Fmoc-AEEA-OH | HATU (2.85 eq) and DIEA (6.00 eq) |
| 36 | Fmoc-γGlu(OtBu)-OH (3.00 eq) | HATU (2.85 eq) and DIEA (6.00 eq) |
| 37 | CO-(CH₂)16-COOH (3.00 eq) | HATU (2.85 eq) and DIEA (6.00 eq) |

### Peptide chain cleavage and purification

1) After the final step of peptide chain synthesis was completed, the resin was washed with MeOH (25.0 mL*3) and vacuum dried. Then the peptide resin was treated with lysis buffer (92.5% TFA/2.5% 3-MPA/2.5% Tis/2.5% H₂O) for 2 hours.
2) The peptide chain was precipitated with an appropriate amount of cold isopropyl ether, centrifuged (3000 rpm for 2 minutes), and washed twice with isopropyl ether. The crude peptide was dried under vacuum for 2 hours.
3) The crude peptide was purified using high-performance liquid chromatography (A: 0.075% TFA in H₂O, B: ACN) to obtain white solid product analogue 1 (122.1 mg, 17.60 µmol, yield 6.29%, purity 97.73%). The obtained analogue was confirmed as analogue 1 using LCMS. The measured molecular weight was 1234.9 [M+4H]⁴⁺, and the theoretical molecular weight was 4935.59.

The intermediates and analogues in the present application are all synthesized according to the above method, and the specific synthesis steps may adopt different combinations of materials and methods to synthesize various corresponding analogues or their salts described in the present application.

### Analogue 2

PKPEK(AEEAc-yGlu-CO(CH₂)₁₆COOH)PE-Aib-DASPEEWQRYYAELRHYLNWLT RQRY-NH₂

Analogue 2 was synthesized using standard Fmoc chemical methods, and the peptide chain synthesis, cleavage, and purification steps were the same as described in Analogue 1.

The crude peptide was purified using high-performance liquid chromatography (A: 0.075% TFA in H₂O, B: ACN) to obtain white solid product PY031 (134 mg, 26.70 µmol, yield 6.67%, purity 95.99%). The obtained analogue was confirmed as Analogue 2 using LCMS. The measured molecular weight was 1227.4[M+4H]⁴⁺, and the theoretical molecular weight was 4905.56.

### Analogue 3

PKPEK(AEEAc-yGlu-CO(CH₂)₁₆COOH)PE-Aib-DASPEEWQRYY-Aib-ELRHYLN WLTRQRY-NH₂

Analogue 3 was synthesized using standard Fmoc chemical methods, and the peptide chain synthesis, cleavage, and purification steps were the same as described in Analogue 1.

The crude peptide was purified using high-performance liquid chromatography (A: 0.075% TFA in H₂O, B: ACN) to obtain white solid product Analogue 3 (127.6 mg, 25.93 µmol, yield 6.48%, purity 95.55%). The obtained analogue was confirmed as analogue 3 using LCMS. The measured molecular weight was 1230.8 [M+4H]⁴⁺, and the theoretical molecular weight was 4919.59.

### Example 2: In vitro activity of PYY₃₋₃₆ analogue

### 1. cAMP assay for determining the in vitro functional activity of PYY analogues on NPY2 receptors

Objective: determining the in vitro functional activity of the PYY analogue from Example 1 compared to natural human PYY₃₋₃₆ by measuring the inhibition of intracellular cAMP production induced by Forskolin in HEK 293 cells overexpressing recombinant human NPY2 receptor.
1) Preparation of analogue: the test analogue was diluted 4-fold to 10 concentration points using 0.5M Tris HCl in an analogue plate using Bravo, and ready for use.
2) Preparation of cells: the cultured cells were collected into a 15 mL sterile centrifuge tube, and centrifuged at 1000 rpm for 5 minutes, with the supernatant being discarded. 10 mL of 1×PBS was added, mixed gently, and centrifuged at 1000 rpm for 5 minutes, with the supernatant being discarded.
3) The cells were re-suspended in detection buffer (20 mM HEPES, 1×HBSS, 0.1% casein, 0.5 mM IBMX), counted with a Vi-cell counter, and diluted to 0.6×10⁶/mL with the detection buffer.
4) According to the arrangement order of Map analogues, 100 nL of prepared analogues were added to a 384-well cell reaction plate using Echo. After addition, they were centrifuged at 1000 rpm for 1 minute.
5) 5 µL of 2 µM Forskolin (prepared with detection buffer) was added to a 384-well cell reaction plate using Multidrop Combi and centrifuged at 1000 rpm for 1 minute. 5 µL of detection buffer containing cells was added to a 384-well reaction plate, ensuring a cell count of 3000 cells per well. It was sealed with transparent sealing film, placed in a room temperature incubator, and incubated for 45 minutes.
6) 10 µL cAMP detection reagent solution (1×cell lysis buffer 9.5 mL, 1×D2-cAMP solution, 1×cAMP-antibody) was added to the corresponding wells of the cell reaction plate using Multidrop Combi. It was covered with a lid and incubated at room temperature in the dark for 1 hour.
7) After incubation for 1 hour, it was placed in an EnVision microplate reader for measurement. The final value was the ratio of OD 665 nm to OD 615 nm.
8) The cAMP value was calculated for each well: % Activity= (signal value of sample well - average signal value of low-signal control well)/( average signal value of high-signal control well - average signal value of low-signal control well ). Using the logarithm of the concentration of analogues as the horizontal axis and the activation rate of cAMP as the vertical axis, the "Nonlinear regression (curve fit)-log (antigen) vs. response-Variable slope" model in GraphPad Prism 5.0 was used for fitting to calculate the EC₅₀ of the sample.

Results:

**Table 1. In vitro cAMP activity of PYY analogues on hNPY2R receptors (average)**

| Analogues | hNPY2R EC₅₀ (nM) |
|---|---|
| PYY₁₋₃₆ | 0.15 |
| PYY₃₋₃₆ | 0.051 |
| Analogue 1 | 0.041 |
| Analogue 2 | 0.037 |
| Analogue 3 | 0.037 |
| Reference Example 1 | 0.044 |

| | |
|---|---|
| Note: Each of the analogues underwent at least 3 independent tests. | |

### 2. Determination of in vitro functional activity of PYY analogues on human NPY1 and Y5 receptors using calcium flux assay.

### Method:

### Day 1: Cell plating

1) Preheated the culture medium, 1×DPBS and 0.05% trypsin EDTA in a 37°C water bath for more than 30 minutes in advance, and ready for use .
2) The preheated culture medium, 1×DPBS and 0.05% trypsin EDTA were taken out, disinfected with 75% alcohol, and placed in a biosafety cabinet.
3) Cultured cells were taken out from the incubator, added with 1 × DPBS, incubated for a moment, suctioned to remove DPBS, added with an appropriate amount of 0.05% trypsin-EDTA for cell digestion, digestion terminated with 10% FBS medium, and dispersed the cells.
4) The dispersed cells were transferred into a 50 mL centrifuge tube using a pipette, centrifuged at 1000 rpm for 5 minutes, resuspended in culture medium, dispersed, and counted.
5) The cells were diluted with culture medium to 1×10⁶ cells/mL, and inoculated at a volume of 20 µL/well of cells onto 384-well poly-Lysine-coated cell plates.
6) The cells were incubated overnight at 37°C in an incubator with 5% CO₂.

### Day 2: FLIPR experiment

### Reagent preparation:

1) Preparation of 250 mM probenecid solution: according to the operating instructions of the kit, 1 mL of FLIPR buffer salt solution was added to 77 mg probenecid.
2) Preparation of 2×(8 µM) Fluo-4 DirectTM sampling buffer: the required number of Fluo-4 Direct^{™} tubes were melted in advance, added with 10 mL of FLIPR buffer salt solution in each tube, added with 0.2 mL of 250 mM probenecid solution, and vortex shaken in the dark for more than 5 minutes.

### Detection methods for analogue:

1) Preparation of test analogue and positive reference agonist: the test analogue was diluted 4-fold to 10 concentrations using Bravo, transferred 900 nL to the analogue plate using Echo, and then added with 30 µL of detection buffer (20 mM HEPES, 1×HBSS, 0.1% casein) to each well. The initial concentration of the test analogue and the positive reference agonist were 1 µM.
2) The cell plate was removed from the incubator (cell Y1 needs to be removed of the culture medium and added with 20 µL of detection buffer, otherwise they will all be a high signal; it is not necessary for Y5), added with 2*Fluo-4 detection reagent, 20 µL per well, incubated in a 37°C incubator for 50 minutes, and then left to stand at room temperature for 10 minutes.
3) The cell plate, the analogue plate, and the pipette tip were placed into the FLIPR instrument, the instrument was started, and 10 µL of analogue was transferred from the analogue plate to the cell plate. In the FLIPR instrument, under the excitation wavelength of 470 nm -496 nm, the calcium ion dye was excited to produce an emission wavelength of 515 nm -575 nm and the fluorescence signal was read.
4) The calcium ion activation rate was calculated for each well: % Activity=( signal value of sample well-average signal value of low-signal control well)/( average signal value of high-signal control well-average signal value of low-signal control well ). Using the logarithm of the concentration of analogues as the horizontal axis and the activation rate of calcium ion fluorescence signal as the vertical axis, the "Nonlinear regression (curve fit)-log (antigen) vs. response Variable slope" model in GraphPad Prism 5.0 was used for fitting to calculate the EC₅₀ of the sample.

Results:

**Table 2. In vitro cAMP activity of PYY analogues on hNPY1R and Y5 receptors (average)**

| Peptide | hNPY1R EC₅₀ (nM) | hNPY5R EC₅₀ (nM) |
|---|---|---|
| PYY₁₋₃₆ | >50 | 7.21 |
| PYY₃₋₃₆ | >50 | 7.81 |
| Analogue 1 | >50 | ND |
| Analogue 2 | >50 | ND |
| Analogue 3 | >50 | 92.73 |

| | | |
|---|---|---|
| Note: Each analogue underwent at least 3 independent tests, with ND indicating not detected. | | |

According to the data in Tables 1 and 2, the PYY₃₋₃₆ analogues of the present application exhibit high selectivity towards NPY2R, but low selectivity towards NPY1R and NPY5R.

### Example 3: In vivo efficacy test of PYY₃₋₃₆ analogues

### 1. Effects on food intake and body weight of normal mice

Objective: comparing the effects of Analogues 1, 2, 3 and Reference Example 1 on weight loss and food intake inhibition in normal mice after a single injection.

### Method:

6-8 weeks old male C57BL/6 mice were fed in animal feeding rooms with strictly controlled environmental conditions, the temperature in the feeding room was maintained at 20°C -24°C and the humidity was maintained at 30%-70%. The light cycle in the animal feeding room was 12 hours (starting at 7:00 am and ending at 7:00 pm). During the experiment, animals were kept in single cages and toys were provided in each cage. During the experiment, animals were free to consume food (growth/reproduction feed for mice and rats) and water.

Body weight without fasting and initial food weight before administration were recorded, and the animals were administered with a single subcutaneous injection, and the daily body weight and food intake weight were recorded for 3 days after administration. The activity, water intake, diet, and body weight changes of animals were observed every day. The changes in body weight and food intake weight were calculated compared to the baseline levels of each group (the initial body weight and food intake weight before administration), with body weight changes expressed as percentages (baseline was 100%).

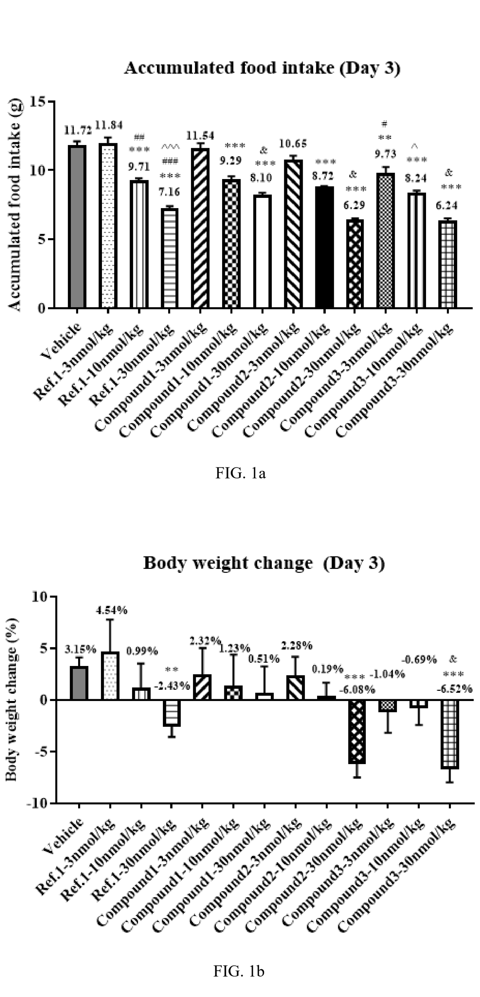

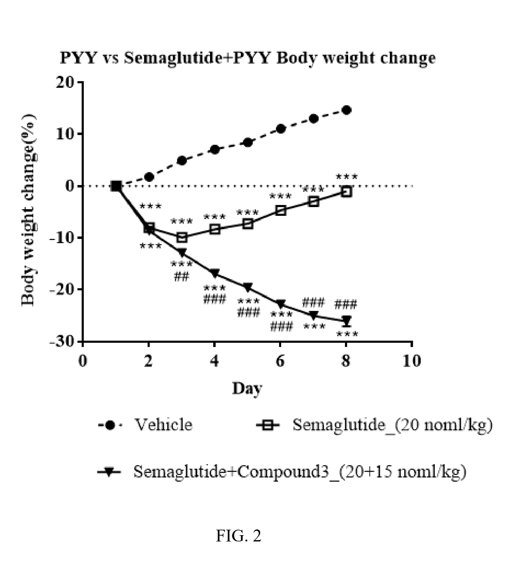

Results: See Table 3, FIG. 1a, and FIG. 1b.

**Table 3: Body weight changes and accumulated food intake weight of C57BL/6 mice over 3 days after a single subcutaneous injection of PYY₃₋₃₆ analogue**

| Peptide | Dose (nmol/kg) | Body weight changes | Accumulated food intake weight |
|---|---|---|---|
| Solvent control | -- | 3.15%±0.90% | 11.72±0.37 g |
| Analogue 1 | 3 | 2.32%±2.53% | 11.54±0.40 g |
| | 10 | 1.23%±2.92% | 9.29±0.27 g |
| | 30 | 0.51%±2.56% | 8.10±0.26 g |
| Analogue 2 | 3 | 2.28%±1.77% | 10.65±0.38 g |
| | 10 | 0.19%±1.39% | 8.72±0.12 g |
| | 30 | -6.08%±1.33% | 6.29±0.21 g |
| Analogue 3 | 3 | -1.04%±2.02% | 9.73±0.48 g |
| | 10 | -0.69%±1.64% | 8.24±0.27 g |
| | 30 | -6.52%±1.37% | 6.24±0.26 g |
| Reference Example 1 | 3 | 4.54%±3.03% | 11.84±0.52 g |
| | 10 | 0.99%±2.37% | 9.71±0.25 g |
| | 30 | -2.43%±1.07% | 7.16±0.22 g |

The data in FIGs. 1a, 1b, and Table 3 strongly support the inhibitory effect of PYY₃₋₃₆ analogues on animal food intake and body weight loss in the present application.

### 2. The pharmacological effects of the combination therapy of PYY₃₋₃₆ analogues and GLP-1 agonist Semaglutide in normal SD rats.

Objective: determining the effect of the combination therapy of PYY₃₋₃₆ analogue and GLP-1 agonist Semaglutide on weight loss and food intake inhibition in normal SD rats.

### Method:

6-8 weeks old male SD rats were fed in animal feeding rooms with strictly controlled environmental conditions, the temperature in the feeding rooms was maintained at 20-24°C and the humidity was maintained at 30%-70%. The light cycle in the animal feeding room was 12 hours (starting at 7:00 am and ending at 7:00 pm). During the experiment, animals were kept in single cages and toys were provided in each cage. During the experiment, animals consume food (growth/reproduction feed for mice and rats) and water freely.

Analogue 3 was tested by subcutaneous administration of a combination of 15 nmol/kg Analogue 3 and 20 nmol/kg GLP-1 agonist Semaglutide (SEQ ID NO: 7) (note: X26 in SEQ ID NO: 7 modified the K side chain through the fatty acid side chain group AEEAc AEEAc-γGlu-CO (CH₂)₁₆COOH), the two drugs were administered separately, once daily for a total of 8 days. during the administration period, the weight changes of animals were monitored daily. The change in weight compared to the baseline level (initial weight before administration) of each group was calculated, and expressed as a percentage (baseline is 100%).

Results: see Table 4 and FIG. 2.

**Table 4 body weight changes in normal SD rats treated with the combination of Analogue 3 and GLP-1 agonist**

| Peptide | Body weight change |
|---|---|
| Solvent control | 14.7%±0.8% |
| Semaglutide (20 nmol/kg) | -1.0%±0.9% |
| Semaglutide (20 nmol/kg) +Analogue 3 (15 nmol/kg) | -26.2%±1.0% |

The data in Table 4 and FIG. 2 indicate that the in vivo efficacy of the PYY₃₋₃₆ analogue of the present application was confirmed by the body weight loss of animals treated with Semaglutide in combination with "Analogue 3" compared to the body weight loss of animals treated with GLP-1 agonist Semaglutide alone.

### Example 4: Pharmacokinetic experiments of PYY₃₋₃₆ analogues

Objective: investigating the pharmacokinetic properties of PYY analogues.

### Method:

Blood samples were collected from each animal at each time point after administration, and the blood samples were centrifuged at around 4°C, 3200 g, for 10 minutes. Plasma was collected separately, transferred to pre-labeled 96-well plates or polypropylene tubes, speedily frozen on dry ice, and stored at -60°C or lower until LC-MS/MS analysis.

LC-MS/MS method was adopted to determine the concentration of the measured analogues in blood samples. The data on the changes in plasma concentration over time were plotted into a chart and analyzed using non partitioned methods with Phoenix WinNonlin 6.3 software program. Relevant PK parameters were calculated based on the administration routes.

### 1. Pharmacokinetics of PYY analogues in SD rats

The plasma pharmacokinetics of PYY analogues in male SD rats were evaluated after a single subcutaneous administration of 0.492 mg/kg Analogue 3. Blood samples were collected from 3 rats at each time point after administration for 0.5h, 1h, 2h, 4h, 8h, 12h, 16h, 24h, 48h, 72h, 96h, 120h, 144h, 168h, extending to 168h. Due to the use of discontinuous sampling to evaluate the kinetics of PYY analogues in rats, the pharmacokinetic parameters of Analogue 3 after a single subcutaneous administration of 0.492 mg/kg were listed using average concentration-time data.

### 2. Pharmacokinetics of PYY analogues in crab-eating macaques

The plasma pharmacokinetics of PYY analogues in male crab-eating macaques were evaluated after a single subcutaneous administration of 0.246 mg/kg Analogue 3. Blood samples were collected from 2 crab-eating macaques at each time point after administration for 1h, 2h, 4h, 8h, 12h, 24h, 48h, 72h, 96h, 120h, 144h, 168h, 192h, 240h, 288h, 336h, 408h, 504h, extending to 504 hours. Due to the use of discontinuous sampling to evaluate the kinetics of PYY analogues in crab-eating macaques, the pharmacokinetic parameters of Analogue 3 after a single subcutaneous administration of 0.246 mg/kg were listed using average concentration-time data.

Results:

**Table 5. Average pharmacokinetic parameters of Analogue 3 after single subcutaneous administration in male rats and male crab-eating macaques**

| Animal | Dose (mg/kg) | T_{1/2} (h) | Tₘₐₓ (h) | Cₘₐₓ (ng/mL) | AUC_{inf} (ng · h/mL) |
|---|---|---|---|---|---|
| Rat | 0.492 | 24.7 | 13.3 | 1941 | 79609 |
| Crab-eating macaque | 0.246 | 125.5 | 12 | 1775 | 336537 |

| | | | | | |
|---|---|---|---|---|---|
| Abbreviation: AUC_{inf}=area under the curve from 0 to infinity; Cₘₐₓ=maximum concentration; Tₘₐₓ=time at maximum concentration; T_{1/2}=half-life. | | | | | |

Table 5 data confirms that the PYY analogues of the present application have a pharmacokinetic profile suitable for once weekly administration.

## Claims

1. A novel peptide tyrosine-tyrosine (PYY₃₋₃₆) analogue or its salt or solvate, comprising a sequence structure of the following general formula (I):
P-K-P-E-ψ-P-E-X₁₀-D-X₁₂-S-P-E-E-W-Q-R-Y-Y-X₂₂-X₂₃-L-R-H-Y-L-N-W-L-T-R-Q-R-Y -R₁ (I) ,
wherein ψ is Lys whose side chain is modified with the structure of the following general formula (II):
Y-Z (II), wherein Y is (AEEAc or Glu)ₐ-(AEEAc or Glu)_{b}-(AEEAc or Glu)_{c}, wherein a, b, and c are each independently 0 or 1, and a, b, and c are not all 0 at the same time, the carboxyl end of Y is connected to the ε-amino group of the side chain of Lys, Z is-CO-(CH₂)ₘ-COOH, and m is an integer between 6-24,
X₁₀ is E or Aib;
X₁₂ is A or Aib;
X₂₂ is A or Aib;
X₂₃ is S or E;
R₁ is NH₂ or OH;
and at least one of X₁₀, X₁₂, and X₂₂ is Aib.

2. The peptide tyrosine-tyrosine (PYY₃₋₃₆) analogue or its salt or solvate according to claim 1, **characterized in that**, Y in general formula (II) is AEEAc-γGlu or AEEAc-AEEAc-γGlu, preferably AEEAc-γGlu.

3. The peptide YY (PYY₃₋₃₆) analogue or its salt or solvate according to claim 1, **characterized in that**, the analogue is selected from:
PKPEK(AEEAc-γGlu-CO(CH₂)₁₆COOH)PEED-Aib-SPEEWQRYY-Aib-SLRHYLNWLT RQRY-NH₂,
PKPEK(AEEAc-γGlu-CO(CH₂)₁₆COOH)PE-Aib-DASPEEWQRYYAELRHYLNWLTRQ RY-NH₂, or
PKPFK(AFFAc-γGlu-CO(CH₂)₁₆COOH)PE-Aib-DASPFFWQRYY-Aib-FLRHYLNWLT RQRY-NH₂.

4. The peptide YY (PYY₃₋₃₆) analogue or its salt or solvate according to claim 1, **characterized in that**, the analogue is selected from PKPEK(AEEAc-γGlu-CO(CH₂)₁₆COOH) PE-Aib-DASPEEWQRYY-Aib-ELRHYLNWLTRQRY-NH₂.

5. A pharmaceutical composition comprising an effective amount of any one of the analogues or its salts or solvates according to claims 1 to 4, and pharmaceutically acceptable excipient, diluent, carrier or pharmaceutic adjuvant.

6. The pharmaceutical composition according to claim 5, **characterized in that**, the form of formulation of the pharmaceutical composition is selected from injection, lyophilized powder, tablet, pill, troche, soft capsule, hard capsule, granule, powder, solution, microneedle, suspension or syrup.

7. The pharmaceutical composition according to claim 5, **characterized in that**, the pharmaceutical composition is in the form of microcapsule, microsphere, nanoparticle, or liposome.

8. The pharmaceutical composition according to claim 5, **characterized in that**, the pharmaceutical composition is used for oral administration, inhalation administration, transdermal administration, or parenteral administration.

9. The pharmaceutical composition according to claim 8, **characterized in that**, the parenteral administration is selected from intraperitoneal, intramuscular, intra-arterial, intravenous, subcutaneous or intradermal injection administration.

10. The pharmaceutical composition according to claim 5, **characterized in that**, the pharmaceutical composition is administered at a frequency of at least once daily or once weekly.

11. Use of an analogue or its salts or solvates according to any one of claims 1 to 4, or a pharmaceutical composition according to any one of claims 5 to 10, for the preparation of a drug for the treatment, prevention, or reduction of overweight and obesity or obesity complications.

12. The use according to claim 11, **characterized in that**, the obesity complications comprise angina pectoris, cardiovascular disease, cholecystitis, cholelithiasis, congestive heart-failure, heart failure with preserved ejection fraction, dyslipidemia, non-alcoholic steatohepatitis, fertility complications, glucose intolerance, gout, hypertension, hypothyroidism, hyperinsulinemia, insulin resistance, osteoarthritis, polycystic ovary syndrome, pregnancy complications, mental derangement, sleep apnea and other respiratory problems, stress urinary incontinence apoplexy, type II diabetes, uric acid kidney stones, mammary cancer, colon cancer, carcinoma of endometrium, esophageal cancer, carcinoma of gallbladder, carcinoma of kidney, prostate cancer or rectal cancer.

13. The use according to claim 11, **characterized in that**, the pharmaceutical composition can be used in combination with other drugs for treating the same or related diseases, the other drugs are selected from one, two or more of metformin, sulfonylurea, SGLT-1/2 inhibitors, DPP-4 inhibitors, insulin, GLP-1, GCG, GIP, and FGF-21.
